# EUROPÄISCHE PATENTSCHRIFT

(11) **EP 0 937 716 B1**
(45) Veröffentlichungstag und Bekanntmachung des Hinweises auf die Patenterteilung: **09.04.2003**
(21) Anmeldenummer: 99810118.2
(22) Anmeldetag: 11.02.1999
(51) Int. Cl.: C07D 251/20, C07D 251/10, A61K 31/53

(54) **Verfahren zur Herstellung von Phenyldichloro-1,3,5-triazinverbindungen**
Process for the preparation of phenyldichloro-1,3,5-triazines
Procédé pour la préparation de phenyldichloro-1,3,5-triazines

(30) Priorität: 20.02.1998 CH 40798
(43) Veröffentlichungstag der Anmeldung: 25.08.1999
(73) Patentinhaber: Ciba Specialty Chemicals Holding Inc., 4057 Basel (CH)
(72) Erfinder: Haase, Jürg, 4126 Bettingen (CH); Mössner, Tanja, 79395 Neuenburg (DE)

(56) Entgegenhaltungen:
- BE-A- 634 399
- US-A- 2 691 020
- US-A- 3 907 721
- US-A- 4 092 466
- BUTLER, ANTHONY R. ET AL: "Reactions of urea, thiourea and related compounds with a number of aldehydes" J. CHEM. RES., SYNOP. (1994), (1), 3 ;ISSN: 0308-2342, XP002104418
- ETIENNE, ANDRE ET AL: "1,3,5-Triazine-2,4-diones" BULL. SOC. CHIM. FR. (1975), (5-6, PT. 2), 1419-24, XP002104419
- SMOLIN; RAPOPORT: "s-Triazines and Derivatives" , INTERSCIENCE PUBLISHERS INC. , NEW YORK XP002104421 * Seite 26 *
- NIGAM ET AL: "Condensation of aldehydes with amides" AGRA UNIVERSITY JOURNAL OF RESEARCH, Bd. VII, Nr. I, 1958, Seiten 67-75, XP002104420

## Beschreibung

Die vorliegende Erfindung betrifft ein einfaches, dreistufiges Verfahren zu Herstellung von Phenyldichloro-1,3,5-triazinverbindungen, ausgehend von Biuret und einer Benzaldehydverbindung.

Phenyldichlorotrazine bzw. ihre Methoxy- und Hydroxyderivate sind z.B. erhältlich durch Benzoylierung von Dicyandiamid, Überführung des Benzoyldicyandiamids in Benzoylbiuret, Cyclisierung dieser Verbindung zu Phenyldihydroxytriazin und anschliessende Chlorierung mit PCl₄ nach folgendem Schema:

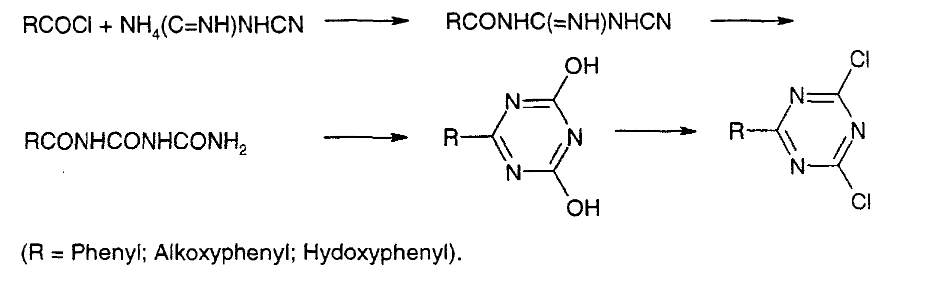

Dieses Herstellungsverfahren ist z.B. aus der US-A-2,691,020 bekannt.

US 4 092 466 A beschreibt die Darstellung von Phenyldichloro-1,3,5-triazinen durch Chlorierung der entsprechenden Phenyldihydroxy-1,3,5-triazine mit Thionylchlorid.

Im Phenylring nicht substituiertes Phenyl-dihydrotriazin (Verbindung der Formel (4), R₁, R₂ = Wasserstoff) lässt sich durch Umsetzung von Biuret mit Benzaldehyd in konzentrierter Schwefelsäure herstellen. Diese Reaktion ist z.B. in Beilst. E III/IV 26, S. 582-583 beschrieben. Die analoge Herstellung der entsprechenden hydroxy- oder alkoxysubstituierten Phenyl-dihydrotriazine gelingt mit diesem Verfahren nicht.

Überraschenderweise wurde nun gefunden, dass diese Verbindungen durch Kondensation von Biuret mit einem substituiertem Phenylaldehyd zu Dioxy-dihydrotriazinen in salzsaurem Medium oder in Anwesenheit von Methylsulfat erhältlich sind.

Das erfindungsgemässe Verfahren zur Herstellung einer Aryl-1,3-5-Triazinverbindung der Formel (1) ist dadurch gekennzeichnet, dass man eine Aldehydverbindung der Formel (2) mit Biuret, Verbindung der Formel (3) in salzsaurem Medium bzw. mit Methylsulfat umsetzt (1. Reaktionsstufe), anschliessend die erhaltene Verbindung der Formel (4) zur Verbindung der Formel (5) bzw. (6) oxidiert (2. Reaktionsstufe) und anschliessend zur Verbindung der Formel (1) chloriert (3. Reaktionsstufe) nach folgendem Schema: 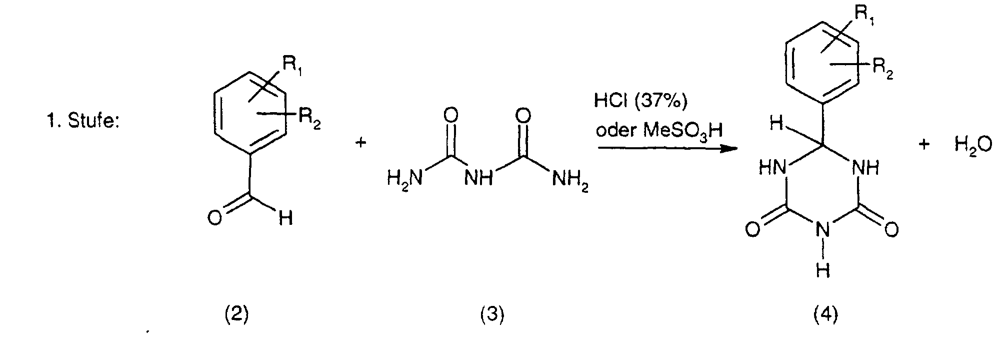 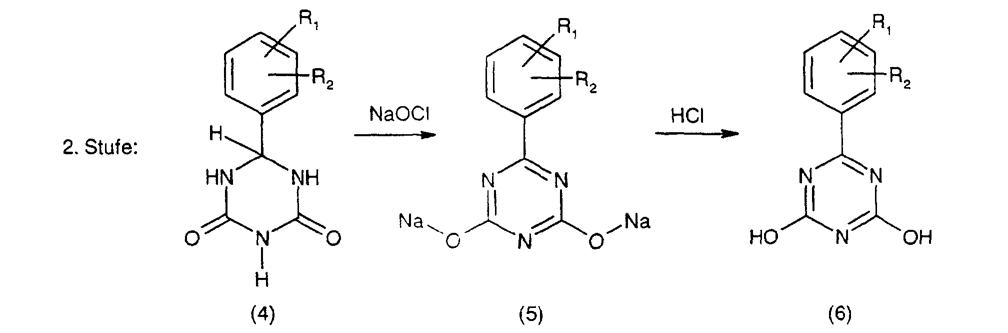 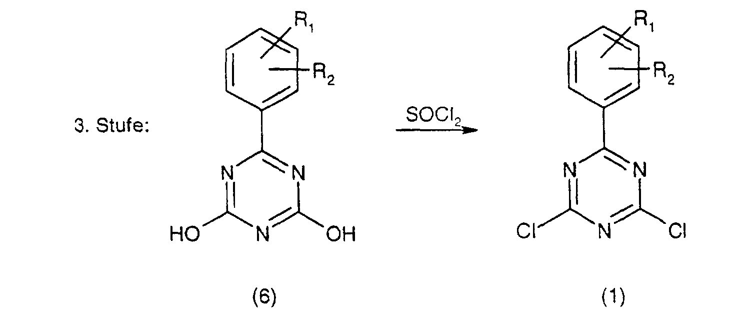 In dem obigen Schema bedeuten
R₁ und R₂ unabhängig voneinander Wasserstoff; Hydroxy oder C₁-C₅-Alkoxy.

C₁-C₅-Alkoxy sind geradkettige oder verzweigte Alkoxyreste wie z.B. Methoxy, Ethoxy, Propoxy, Butoxy, oder Pentyloxy.

Vorzugsweise betrifft das erfindungsgemässe Verfahren die Herstellung von Verbindungen der Formel (1), worin
- R₁: C₁-C₅-Alkoxy; und
- R₂: Wasserstoff bedeuten, und ganz besonders Verbindungen der Formel (1), worin
- R₁: Methoxy bedeutet.

Die Reaktionszeit für die erste Reaktionsstufe beträgt in der Regel 1 bis 24, vorzugsweise 5 bis 12 Stunden. Die Reaktion wird bei Temperaturen von 0 bis 60°C, und vorzugsweise bei Raumtemperatur durchgeführt.

Die Durchführung der ersten Reaktionsstufe erfolgt im allgemeinen so, dass zunächst die Salzsäure, bzw. MeSO₃H vorgelegt wird, in diese ca. 1 mol Biuret eingerührt und 1 mol der Verbindung der Formel (2) zudosiert wird. Anschliessend gibt man noch etwa 0,2 mol Biuret und Salzsäure bzw. MeSO₃H hinzu und rührt die Reaktionsmasse innerhalb von 1 bis 8 Stunden aus.

Lösungsmittel und weitere Zusätze sind für die erste Reaktionsstufe im allgemeinen nicht erforderlich. Gegebenenfalls können als Lösungsmittel einwertige Alkohole, wie z.B. Methanol, Ethanol oder i-Propanol hinzugefügt werden. Auch auf die Verwendung von Katalysatoren kann verzichtet werden.

Im zweiten Reaktionsschritt wird die Verbindung der Formel (4) in wässriger Suspension mit NaOCI zur Verbindung der Formel (6) oxidiert. Die Reaktionsmasse wird mit HCI versetzt. Die freigesetzte Cyanursäure (Verbindung der Formel (6)) wird isoliert und getrocknet.

In der dritten Reatkionsstufe wird mit Thionylchlorid und in Anwesenheit von DMF zur Verbindung der Formel (1) chloriert.

Die nach dem erfindungsgemässen Verfahren hergestellten Verbindungen der Formel (1) finden Verwendung als UV-Absorber oder sind Ausgangsprodukte für die Herstellung von UV-Absorbern. 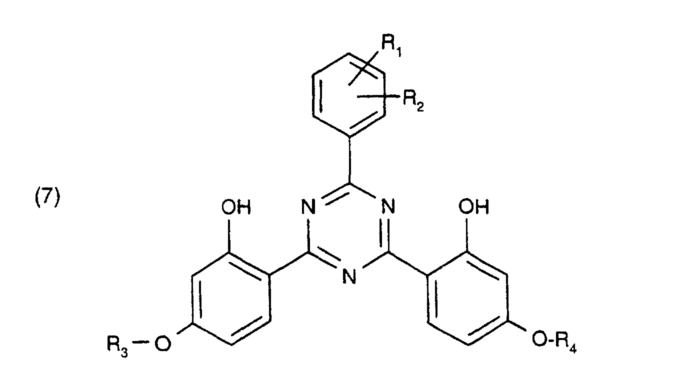 herstellen.

In Formel (7) bedeuten
- R₁ und R₂: unabhängig voneinander Wasserstoff; Hydroxy oder C₁-C₅-Alkoxy;
- R₃ und R₄: unabhängig voneinander, C₃-C₁₈-Alkyl; C₂-C₁₈-Alkenyl; einen Rest der Formel -CH₂-CH(-OH)-CH₂-O-T₁ ;
einen Rest der Formel (7a) 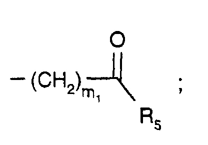
R₅ Hydroxy; nicht substituiertes oder durch ein oder mehrere OH-Gruppen substituiertes C₁-C₅-Alkoxy; Amino; Mono- oder Di-C₁-C₅-Alkylamino; M; einen Rest der Formel 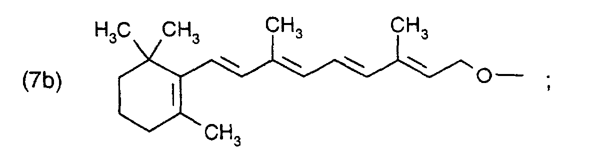 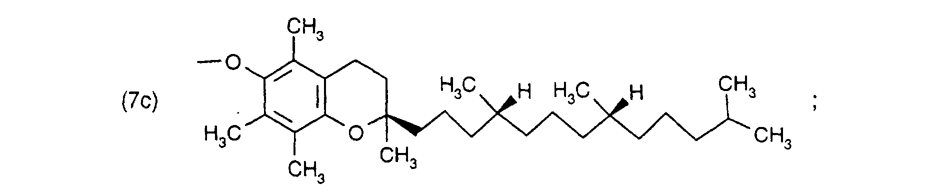 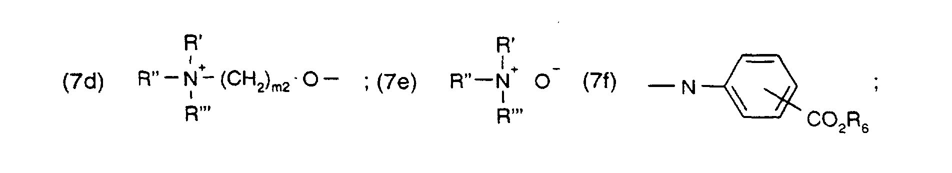 worin R', R" und R"' unabhängig voneinander nicht substituiertes oder durch ein oder mehrere OH-Gruppen substituiertes C₁-C₁₄-Alkyl;
- R₆: Wasserstoff; M; C₁-C₅-Alkyl; oder einen Rest der Formel -(CH₂)_{m₃}-O-T₁ ;
- T₁: Wasserstoff; oder C₁-C₈-Alkyl;
- M: Alkalimetall; und
- m₁, m₂ und m₃: unabhängig voneinander 1 bis 4 bedeuten.

Die beiden Resorcingruppen 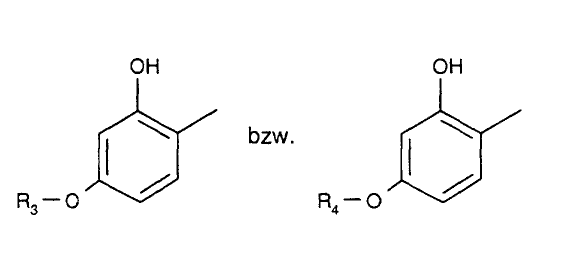 werden in allgemein bekannter Weise durch Friedel-Crafts-Acylierung von Resorcin in Gegenwart einer Lewis-Säure, insbesondere Aluminiumchlorid, eingeführt. In der letzten Stufe erfolgt die Veretherung der freien, p-ständigen Hydroxylgruppen, je nach Bedeutung der Reste R₃ und R₄, durch Alkylierung bzw. säurekatalysierte Addition von Glycidylethern. Die Verbindungen der Formel (7) eignen sich insbesondere als UV-Filter, d.h. zum Schützen von ultraviolett empfindlichen organischen Materialien, insbesondere der Haut und Haare von Menschen und Tieren vor der schädigenden Einwirkung von UV-Strahlung. Diese Verbindungen eignen sich daher als Lichtschutzmittel in kosmetischen, pharmazeutischen und veterinärmedizinischen Präparaten. Diese Verbindungen können sowohl gelöst als auch im mikronisierten Zustand verwendet werden.

Für die kosmetische Verwendung haben diese Lichtschutzmittel gewöhnlich eine mittlere Partikelgrösse im Bereich von 0,02 bis 2, vorzugsweise 0,05 bis 1,5, und ganz besonders von 0,1 bis 1,0 µ. Die unlöslichen erfindungsgemässen UV-Absorber können durch übliche Methoden, z.B. Mahlen mit z.B. einer Düsen-, Kugel-, Vibrations- oder Hammermühle auf die gewünschte Partikelgrösse gebracht werden. Vorzugsweise wird das Mahlen in Anwesenheit von 0,1 bis 30, vorzugsweise 0,5 bis 15 Gew.-%, bezogen auf den UV-Absorber, einer Mahlhilfe wie z.B. eines alkylierten Vinylpyrrolidon-Polymers, eines Vinylpyrrolidon-Vinylacetat-Copolymers, eines Acylglutamates oder insbesondere eines Phospholipids durchgeführt.

Die kosmetische Zusammensetzung kann neben den erfindungsgemässen UV-Absorbern auch noch einen oder mehrere weitere UV-Schutzstoffe, wie z.B. einen organischen UV-Absorber aus der Klasse der p-Aminobenzoesäurederivate, Salicylsäurederivate, Benzophenonderivate, Dibenzoylmethanderivate, Diphenylacrylatderivate, Benzofuranderivate, der polymeren UV-Absorber, enthaltend eine oder mehrere silizium-organsiche Reste, Zimtsäurederivate, Campherderivate, Trianilino-s-Triazinderivate, Phenylbenzimidazolsulfonsäure und deren Salze, Menthyl-Anthranilate, Benzotriazolderivate, und/oder ein anorganisches Mikropigment ausgewählt aus mit Aluminiumoxid oder Siliciumdioxid umhülltem TiO₂, Zinkoxid oder Mica.

Die kosmetischen Zusammensetzungen enthalten 0,1 bis 15, vorzugsweise 0,5 bis 10 Gew.-%, bezogen auf das Gesamtgewicht der Zusammensetzung, eines UV-Absorbers oder eines Gemisches aus UV-Absorbern und einen kosmetisch verträglichen Hilfsstoff.

Die Herstellung der kosmetischen Zusammensetzungen kann durch physikalisches Mischen des oder der UV-Absorber mit dem Hilfsstoff durch gewöhnliche Methoden, wie z.B. durch einfaches Zusammenrühren der Einzelkomponenten erfolgen.

Die kosmetischen Zusammensetzungen können als Wasser-in-Öl- oder Öl-in-Wasser-Emulsion, als Öl-in-Alkohol-Lotion, als vesikulare Dispersion eines ionischen oder nichtionischen amphiphilen Lipids, als Gel, fester Stift oder als Aerosol-Formulierung formuliert werden.

Als Wasser-in-Öl- oder Öl-in-Wasser-Emulsion enthält der kosmetisch verträgliche Hilfs stoff vorzugsweise 5 bis 50% einer Ölphase, 5 bis 20% eines Emulgators und 30 bis 90% Wasser. Die Ölphase kann dabei irgendein für kosmetische Formulierungen geeignetes Öl enthalten, wie z.B. ein oder mehrere Kohlenwasserstofföle, ein Wachs, ein natürliches Öl, ein Silikonöl, einen Fettsäureester oder einen Fettalkohol. Bevorzugte Mono- oder Polyole sind Ethanol, Isopropanol, Propylenglykol, Hexylenglycol, Glycerin und Sorbitol.

Für die kosmetischen Zusammensetzungen kann jeder konventionell einsetzbare Emulgator verwendet werden, wie z.B. einer oder mehrere ethoxylierte Ester von natürlichen Derivaten, wie z.B. polyethoxylierte Ester von hydrogeniertem Castor-ÖI; oder ein Silikonöl-Emulgator wie z.B. Silikonpolyol; eine gegebenenfalls ethoxylierte Fettsäureseife; ein ethoxylierter Fettalkohol; ein gegebenenfalls ethoxylierter Sorbitanester; eine ethoxylierte Fettsäure; oder ein ethoxyliertes Glycerid.

Die kosmetischen Zusammensetzungen können auch weitere Komponenten, wie z.B. Emollients, Emulsionsstabilisatoren, Haut-Feuchthaltemittel, Hautbräunungsbeschleuniger, Verdickungsmittel wie z.B. Xanthan, Feuchtigkeit-Retentionsmittel wie z.B. Glycerin, Konservierungsmittel, Duft- und Farbstoffe enthalten.

Die kosmetischen Formulierungen zeichnen sich durch exzellenten Schutz der menschlichen Haut gegen den schädigenden Einfluss von Sonnenlicht aus.

In den folgenden Beispielen beziehen sich die Prozentsätze auf das Gewicht. Die Mengen beziehen sich auf die Reinsubstanz.

### Beispiel 1: Herstellung von 4,6-Dioxy-2-(4-Methoxyphenyl)-1,2-dihydro-1,3,5-triazin

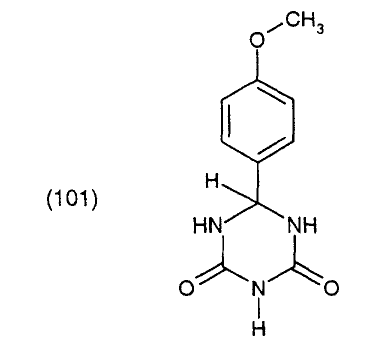

In einem Planschliffkolben werden 951 g 37%ige Salzsäure vorgelegt, 105,1 g Biuret eingerührt und bei Raumtemperatur 136,15 g (1 mol) Anisaldehyd zudosiert. Die Suspension wird während 18 Stunden verrührt. Hierauf werden 18,5 g Biuret und 95 g 37%ige Salzsäure nachgesetzt und die Reaktionsmasse während 4 Stunden ausgerührt. Der Reaktorinhalt wird auf 3000 ml Eiswasser ausgetragen und über eine Nutsche filtriert. Der Filterkuchen wird mit viel Wasser gewaschen und im Vakuum getrocknet.
Ausbeute: 214 g der Verbindung der Formel (101)

### Beispiele 2 bis 6:

Analog Beispiel 1 erhält man die in Tabelle 1 aufgeführten Verbindungen der Formeln (102) bis (106).

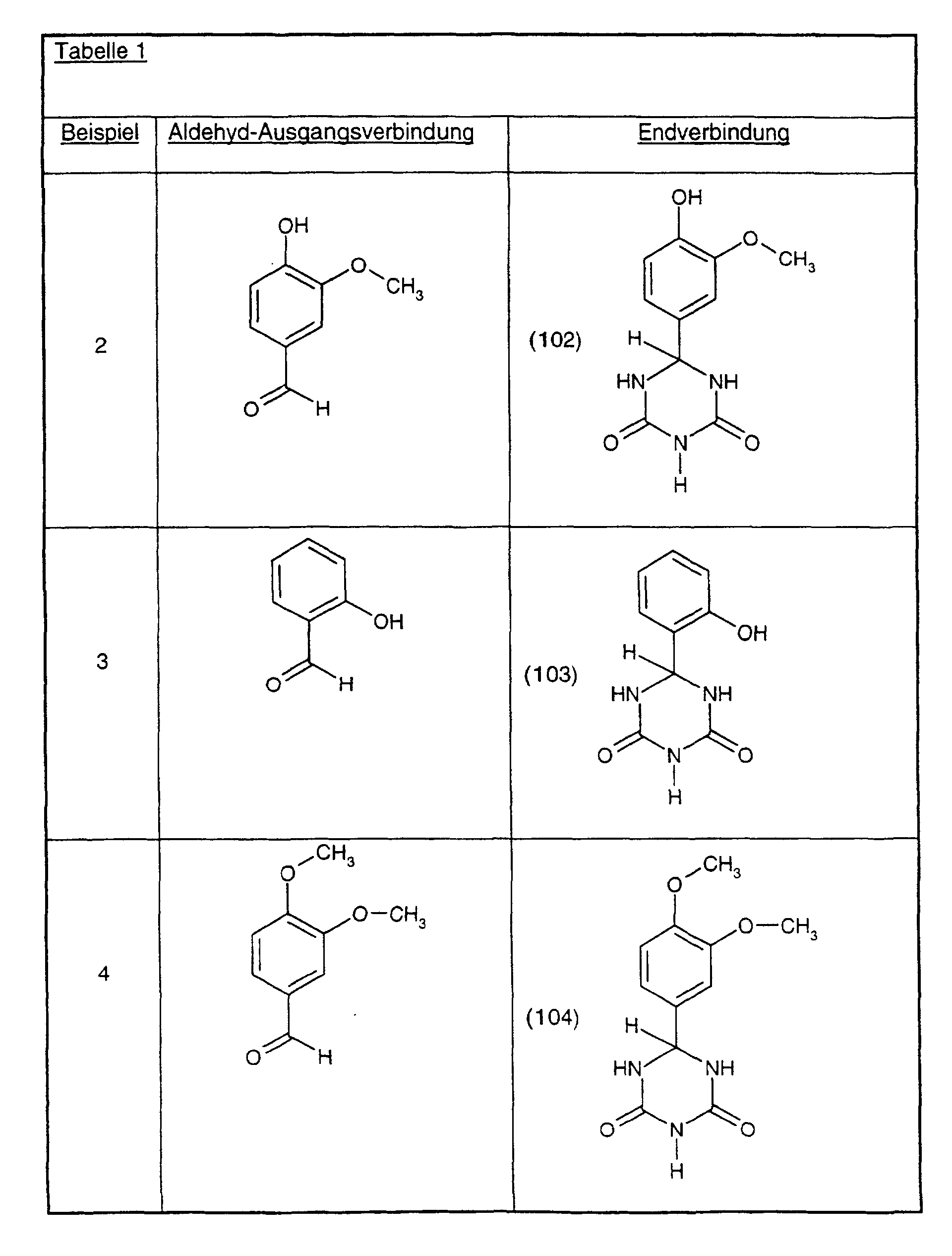

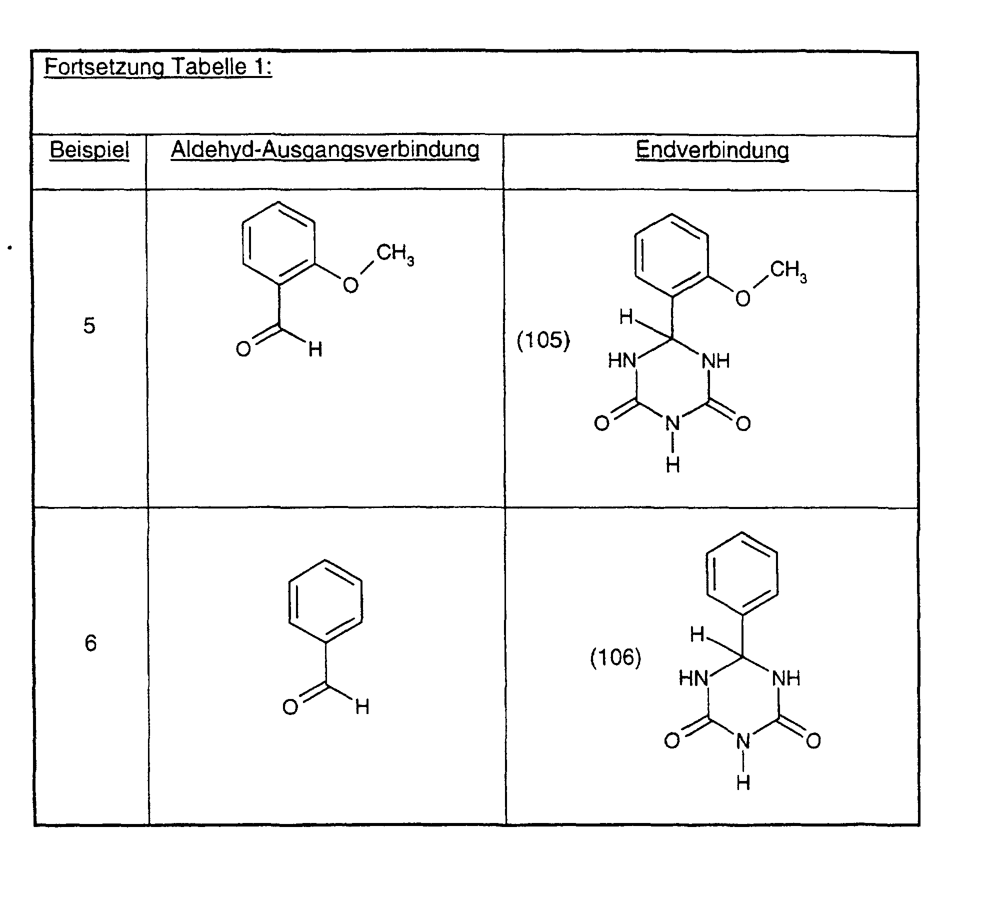

## Patentansprüche

1. Verfahren zur Herstellung einer Aryl-1,3-5-Triazinverbindung der Formel (1), **dadurch gekennzeichnet, dass** man eine Aldehydverbindung der Formel (2) mit Biuret, Verbindung der Formel (3), in salzsaurem Medium bzw. mit Methylsulfat umsetzt (1. Reaktionsstufe), anschliessend die erhaltene Verbindung der Formel (4) zur Verbindung der Formel (5) bzw. (6) oxidiert (2. Reaktionsstufe) und anschliessend zur Verbindung der Formel (1) chloriert (3. Reaktionsstufe) nach folgendem Schema: 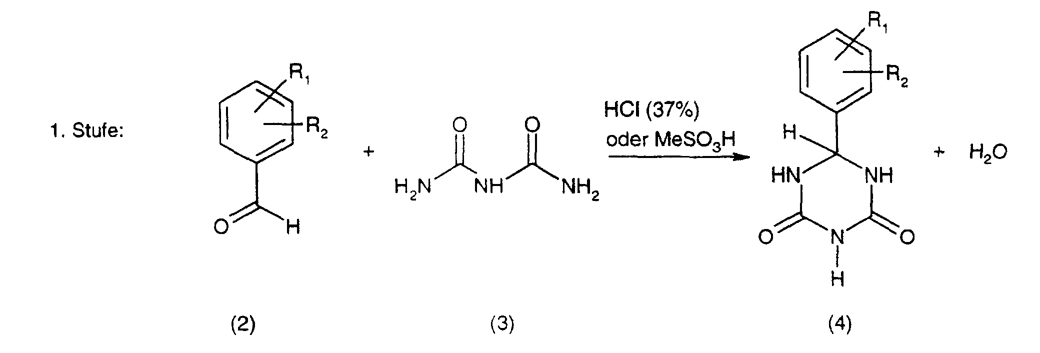 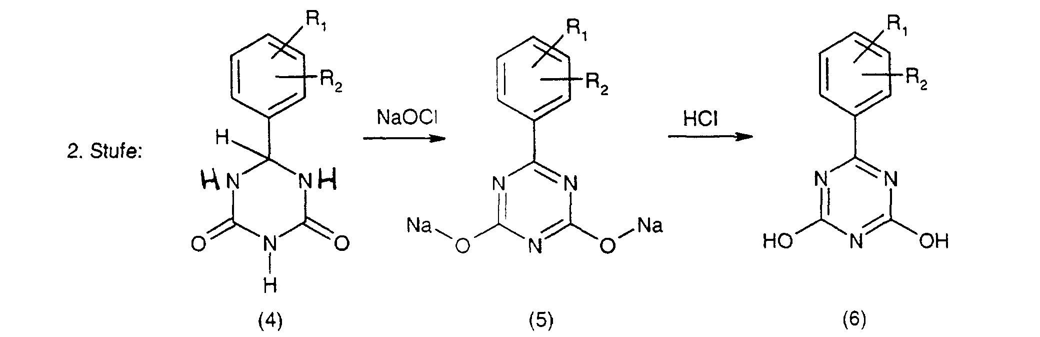 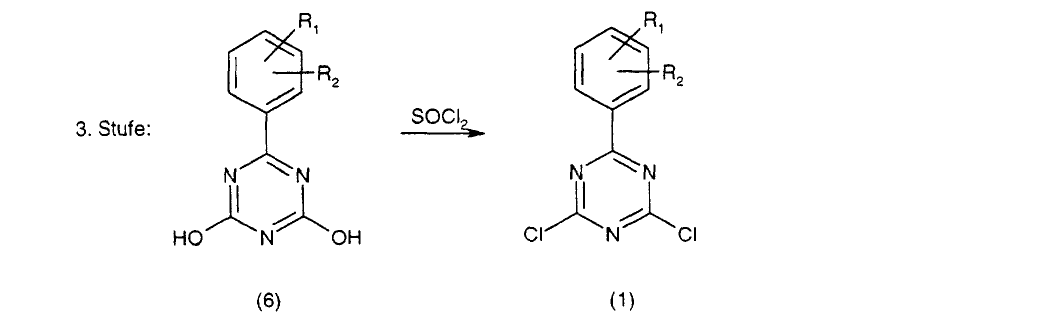 worin in dem obigen Schema
R₁ und R₂ unabhängig voneinander Wasserstoff; Hydroxy oder C₁-C₅-Alkoxy;
bedeuten.

2. Verfahren nach Anspruch 1, **dadurch gekennzeichnet, dass** die Reaktionszeit der ersten Stufe 1 bis 24 Stunden beträgt.

3. Verfahren nach Anspruch 1 oder 2, **dadurch gekennzeichnet, dass** die erste Reaktionsstufe bei einer Temperatur von 0 bis 60°C durchgeführt wird.

4. Verfahren nach einem der Ansprüche 1 bis 3, **dadurch gekennzeichnet, dass** die erste Reaktionsstufe bei Raumtemperatur durchgeführt wird.

5. Verfahren nach einem der Ansprüche 1 bis 4, **dadurch gekennzeichnet, dass**
R₁ C₁-C₅-Alkoxy; und
R₂ Wasserstoff bedeuten.

6. Verfahren nach Anspruch 5, **dadurch gekennzeichnet, dass**
R₁ Methoxy bedeutet.

## Claims

1. A process for the preparation of an aryl-1,3-5-triazine compound of formula (1), which comprises reacting an aldehyde compound of formula (2) with biuret, compound of formula (3), in hydrochloric medium or with methyl sulfate (1st reaction step), then oxidising the resulting compound of formula (4) to the compound of formula (5) or (6) (2nd reaction step) and subsequently chorinating the compound so obtained to the compound of formula (1) (3rd reaction step) according to the following scheme: 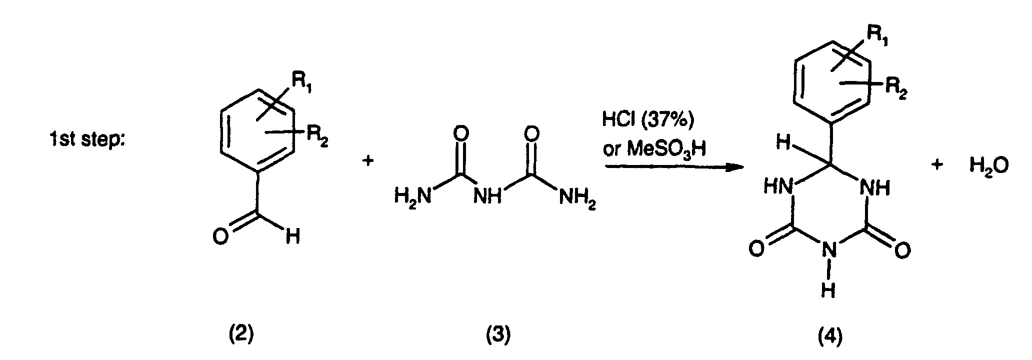 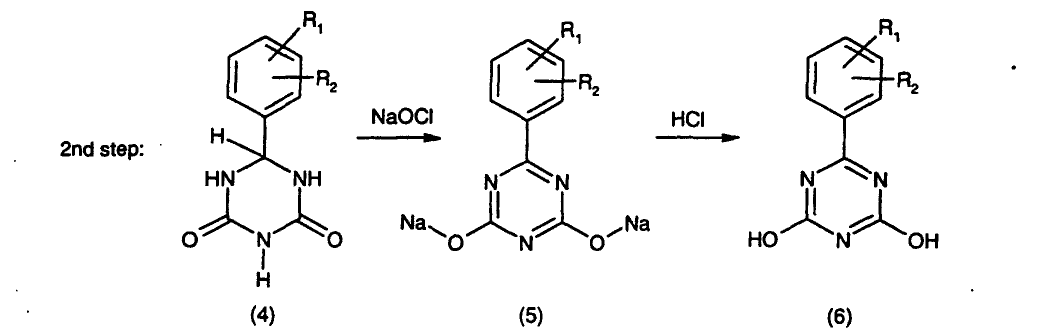 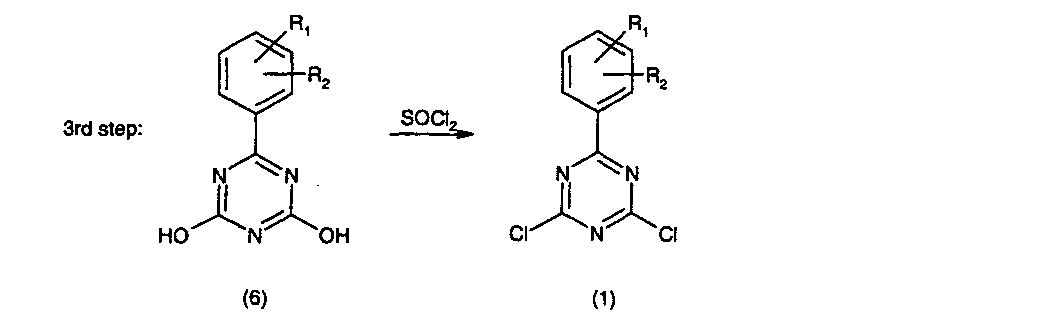 in which above scheme
R₁ and R₂ are each independently of the other hydrogen; hydroxy or C₁-C₅alkoxy.

2. A process according to claim 1, wherein the reaction time of the first reaction step is from 1 to 24 hours.

3. A process according to either claim 1 or claim 2, wherein the first reaction step is carried out at a temperature from 0 to 60°C.

4. A process according to any one of claims 1 to 3, wherein the first reaction step is carried out at room temperature.

5. A process according to any one of claims 1 to 4, wherein
R₁ is C₁-C₅alkoxy; and
R₂ is hydrogen.

6. A process according to claim 5, wherein
R₁ is methoxy.

## Revendications

1. Procédé pour la préparation d'un composé aryl-1,3,5-triazine de formule (1), **caractérisé en ce que** l'on met à réagir un composé aldéhyde de formule (2) avec du biuret, un composé de formule (3), en milieu acide chlorhydrique ou avec du sulfate de méthyle (étape de réaction 1), on oxyde ensuite le composé obtenu de formule (4) en composé de formule (5) ou (6) (étape de réaction 2) et on réalise une chloration ensuite pour obtenir le composé de formule (1) (étape de réaction 3) selon le schéma suivant : 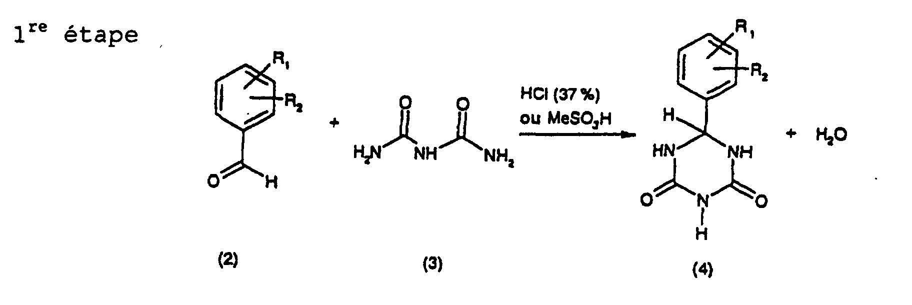 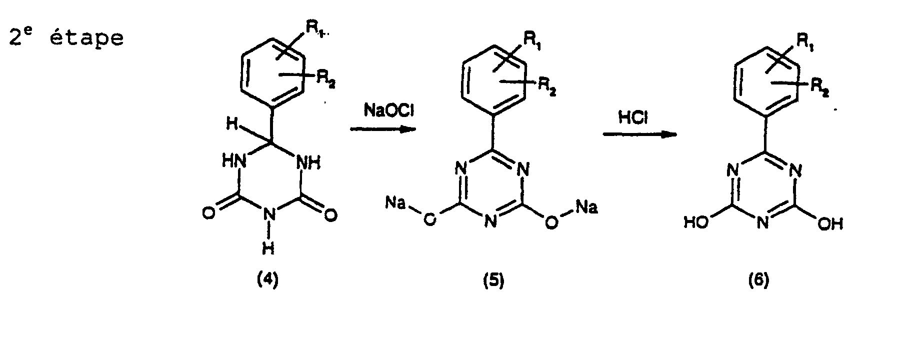 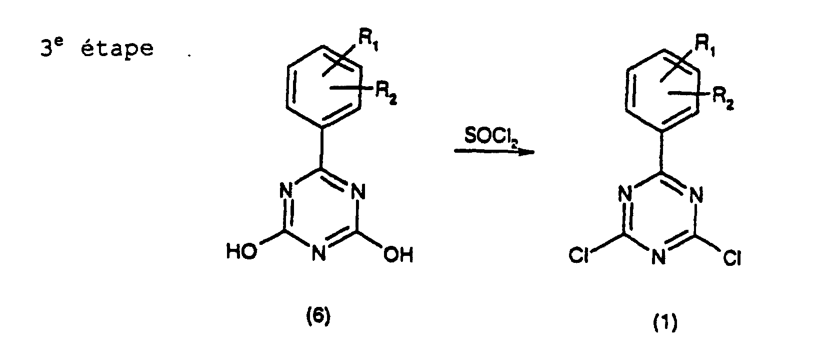 dans lequel dans le schéma susmentionné
R₁ et R₂ représentent indépendamment l'un de l'autre un atome d'hydrogène ; un groupe hydroxy ou alcoxy en C₁ à C₅.

2. Procédé selon la revendication 1, **caractérisé en ce que** le temps de réaction de la première étape s'élève de 1 à 24 heures.

3. Procédé selon la revendication 1 ou 2, **caractérisé en ce que** la première étape de réaction est réalisée à une température de 0 à 60 °C.

4. Procédé selon une des revendications 1 à 3, **caractérisé en ce que** la première étape de réaction est réalisée à température ambiante.

5. Procédé selon une des revendications 1 à 4, **caractérisé en ce que**
R₁ représente un groupe alcoxy en C₁ à C₅ ; et
R₂ représente un atome d'hydrogène.

6. Procédé selon la revendication 5, **caractérisé en ce que**
R₁ représente un groupe méthoxy.
